# EUROPEAN PATENT APPLICATION

(11) **EP 3 828 291 A1**
(43) Date of publication of application: **02.06.2021**
(21) Application number: 19842340.2
(22) Date of filing: 19.07.2019
(51) Int. Cl.: C12Q 1/6886, C12N 15/11, G01N 33/574

(54) **METHYLATION MODIFICATION-BASED TUMOR MARKER STAMP-EP1**

(30) Priority: 26.07.2018 CN 201810830893
(71) Applicant: Shanghai Epiprobe Biotechnology Co., Ltd., Shanghai 200233 (CN)
(72) Inventor: LI, Zhenyan, Shanghai 200233 (CN)
(74) Representative: IPLodge bv
(86) International application number: PCT/CN2019/096793
(87) International publication number: WO 2020/020071

(57) **Abstract**

A methylated tumor marker STAMP-EP1 and a use thereof, which relate to the field of molecular biology. The present disclosure further relates to a use of the methylated tumor marker STAMP-EP1 in the preparation of tumor diagnostic agents. The tumor marker STAMP-EP1 herein is hypermethylated in all tumor types, is hypomethylated in corresponding normal tissue, and with very high sensitivity and specificity; primers for detecting STAMP-EP1 may be used to prepare a tumor diagnostic kit.

## Description

### FIELD OF DISCLOSURE

The disclosure is in the field of disease diagnostic markers. More specifically, the disclosure relates to a methylation based tumor marker STAMP, Specific Tumor Aligned Methylation of Pan-cancer.

### BACKGROUND OF DISCLOSURE

The occurrence and development of tumor is a complex, multi-level, multi-factor dynamic process, including the complex interaction of external environment, genetic variations and epigenetic changes, etc. Environmental factors include carcinogenic physical, chemical, biological and other factors as well as unhealthy living habits. Genetic variations include gene mutation, copy number variation, chromosome translocation and so on. Epigenetic changes mainly include DNA methylation, histone modification, non-coding RNA and other factors. In the process of tumor occurrence and development, environmental, genetic and epigenetic factors complement each other and act together, leading to a series of inactivations of tumor suppressor genes and activations of proto-oncogenes, thereby causing tumor. The three factors act throughout the development of tumor and interact with each other. For human beings, there are still many challenges for tumor therapy at present. Although new surgical methods, targeted therapy and immunotherapy have made some gratifying progress in recent years, there are still many misconceptions about tumor. The major problems of tumor metastasis, recurrence, heterogeneity and drug resistance need to be solved urgently.

There are many types of tumors in human body, and the occurrences of tumors can be found in most of the tissues. Different types of tumors can be divided into many subtypes. Especially in recent years, the classification of tumors is more detailed due to the progress in tumor molecular biology. For different types, different stages or different molecular subtypes of tumors, the therapeutic regimes are also different.

With the deepening of the understanding in tumor and the progress of science and technology, many new tumor markers have been found and used in clinical diagnosis. Before 1980, tumor markers were mainly hormones, enzymes, proteins and other cell secretions, such as carcinoembryonic antigen (CEA) and alpha fetoprotein (AFP) used as markers of gastric cancer, liver cancer and other tumors, carbohydrate antigen 125 (CA125) used as a marker of cervical cancer, and prostate specific antigen (PSA) used as a marker of prostate cancer. Although these tumor markers are still used in clinic, their sensitivity and accuracy can not meet the clinical needs.

Fluid biopsy is a technique for the diagnosis and prediction of tumors using circulating tumor cells or circulating tumor DNA as detection targets. The technology is still in its infancy, having many shortcomings. First, the sensitivity and specificity are not good enough. The tumor itself is heterogeneous, including a variety of subtypes of cell populations. The proportion of tumor DNA in clinical samples, especially blood samples, is very low. The existing tumor markers are difficult to meet the sensitivity of clinical requirements, and it is easy to cause misdiagnosis. Second, one marker has good effect only for one or a few kinds of tumors. As the DNA in blood are very complex, the existing tumor markers cannot solve the complex problems of tumor source and metastasis. Because of these complexities, it is difficult for many DNA methylation tumor markers to have a unified standard in clinical application, which seriously affects the sensitivity and accuracy of the markers. Human tumors have both characteristics and commonness. A common marker for different tumors is of great significance for tumor screening, diagnosis, treatment and efficacy evaluation.

Therefore, it is urgent to develop new tumor markers with general applicability, high accuracy and allowing easy judgment in tumor diagnosis.

### SUMMARY OF DISCLOSURE

The object of the disclosure is to provide a method for detecting tumor based on abnormal hypermethylation of specific sites in tumor using DNA methylation modification as tumor marker.

The first aspect of the present disclosure provides an isolated polynucleotide, including: (a) a polynucleotide with a nucleotide sequence as shown in SEQ ID NO: 1; (b) a fragment of the polynucleotide of (a), having at least one (such as 2-178, more specifically 3, 5, 10, 15, 20, 25, 30, 50, 60, 80, 100, 120, 150) CpG site with modification; and/or (c) a nucleic acid (such as the polynucleotide with a nucleotide sequence as shown in SEQ ID No: 3) complementary to the polynucleotide or fragment of (a) or (b).

In a preferable embodiment, said modification includes 5-methylation, 5-hydroxymethylation, 5-aldehyde methylation or 5-carboxymethylation.

The second aspect of the disclosure provides an isolated polynucleotide, which is converted from the polynucleotide of the first aspect, and as compared with the sequence of the first aspect, the cytosine C of the CpG site(s) with modification is unchanged, and the unmodified cytosine is converted into T or U.

In a preferable embodiment, it is converted from the polynucleotide corresponding to the first aspect by bisulfite treatment. In another preferable embodiment, the polynucleotide includes: (d) a polynucleotide with a nucleotide sequence as shown in SEQ ID NO: 2 or 4; (e) a fragment of the polynucleotide of (d), having at least one (such as 2-178, more specifically 3, 5, 10, 15, 20, 25, 30, 50, 60, 80, 100, 120, 150) CpG site with modification.

The third aspect of the disclosure provides a use of the polynucleotide described in the first or second aspect in manufacture of a tumor detection agent or kit.

In a preferable embodiment, the tumors include (but are not limited to): hematologic cancers such as leukemia, lymphoma, multiple myeloma; digestive system tumors such as esophageal cancer, gastric cancer, colorectal cancer, liver cancer, pancreatic cancer, bile duct and gallbladder cancer; respiratory system tumors such as lung cancer, pleuroma; nervous system tumors such as glioma, neuroblastoma, meningioma; head and neck tumors such as oral cancer, tongue cancer, laryngeal cancer, nasopharyngeal cancer; gynecological and reproductive system tumors such as breast cancer, ovarian cancer, cervical cancer, vulvar cancer, testicular cancer, prostate cancer, penile cancer; urinary system tumors such as kidney cancer, bladder cancer, skin and other systems tumors such as skin cancer, melanoma, osteosarcoma, liposarcoma, thyroid cancer.

In another preferable embodiment, samples of the tumor include but are not limited to: tissue samples, paraffin embedded samples, blood samples, pleural effusion samples, and alveolar lavage fluid samples, ascites and lavage fluid samples, bile samples, stool samples, urine samples, saliva samples, sputum samples, cerebrospinal fluid samples, cell smear samples, cervical scraping or brushing samples, tissue and cell biopsy samples.

The fourth aspect of the disclosure provides a method of preparing a tumor detection agent, including: providing the polynucleotide described in the first or second aspect, designing a detection agent for specifically detecting the modification on CPG site(s) of a target sequence which is the full length or fragment of the polynucleotide; wherein, the target sequence has at least one (such as 2-178, more specifically, 3, 5, 10, 15, 20, 25, 30, 50, 60, 80, 100, 120, 150) modified CpG site; preferably, the detection agent includes (but is not limited to) primers or probes.

The fifth aspect of the disclosure provides an agent or a combination of agents which specifically detect the modification on CPG site(s) of a target sequence, which is the full length or fragment of any of the polynucleotides described in the first or second aspect and has at least one (such as 2-178, more specifically, 3, 5, 10, 15, 20, 25, 30, 50, 60, 80, 100, 120, 150) modified CpG site.

In a preferable embodiment, the agent or combination of agents is for a gene sequence containing the target sequence (designed based on the gene sequence), and the gene sequence includes gene Panels or gene groups.

In another preferable embodiment, the detection agent comprises: primers or probes.

In another preferable embodiment, the primers are: the primers shown in SEQ ID NO: 3 and 4; the primers shown in SEQ ID NO: 7 and 8; the primers shown in SEQ ID NO: 9 and 10; the primers shown in SEQ ID NO: 11 and 12; or the primers shown in SEQ ID NO: 13 and 14.

In the sixth aspect of the disclosure, a use of the agent or combination of agents described in the fifth aspect of the disclosure in the manufacture of a kit for detecting tumors is provided; preferably, the tumors include (but are not limited to): digestive system tumors such as esophageal cancer, gastric cancer, colorectal cancer, liver cancer, pancreatic cancer, bile duct and gallbladder cancer; respiratory system tumors such as lung cancer, pleuroma; hematologic cancers such as leukemia, lymphoma, multiple myeloma; gynecological and reproductive system tumors such as breast cancer, ovarian cancer, cervical cancer, vulvar cancer, testicular cancer, prostate cancer, penile cancer; nervous system tumors such as glioma, neuroblastoma, meningioma; head and neck tumors such as oral cancer, tongue cancer, laryngeal cancer, nasopharyngeal cancer; urinary system tumors such as kidney cancer, bladder cancer, skin and other systems tumors such as skin cancer, melanoma, osteosarcoma, liposarcoma, thyroid cancer.

The seventh aspect of the present disclosure provides a detection kit, comprising container(s) and the agent or combination of agents described above in the container(s); preferably, each agent is placed in an independent container.

In a preferable embodiment, the kit also includes: bisulfite, DNA purification agent, DNA extraction agent, PCR amplification agent and/or instruction for use (indicating operation steps of the detection and a result judgment standard).

In the eighth aspect of the disclosure, a method for detecting the methylation profile of a sample *in vitro* is provided, including: (i) providing the sample and extracting the nucleic acid; (ii) detecting the modification on CPG site(s) of a target sequence in the nucleic acid of (i), wherein the target sequence is the polynucleotide described in the first aspect or the polynucleotide converted therefrom,which described in the second aspect.

In a preferable embodiment, in step (3), the analysis methods include pyrosequencing, bisulfite conversion sequencing, method using methylation chip, qPCR, digital PCR, second generation sequencing, third generation sequencing, whole genome methylation sequencing, DNA enrichment detection, simplified bisulfite sequencing technology, HPLC, MassArray, methylation specific PCR (MSP), or their combination, as well as *in vitro* detection and *in vivo* tracer detection for the combined gene group of partial or all of the methylation sites in the sequence shown in SEQ ID NO: 1. In addition, other methylation detection methods and newly developed methylation detection methods in the future can be applied to the disclosure.

In another preferable embodiment, step (ii) includes: (1) treating the product of (i) to convert the unmodified cytosine into uracil; preferably, the modification includes 5-methylation, 5-hydroxymethylation, 5-aldehyde methylation or 5-carboxymethylation; preferably, treating the nucleic acid of step (i) with bisulfite; and (2) analyzing the modification of the target sequence in the nucleic acid treated by (1).

In another preferable embodiment, the abnormal methylation profile is the high level of methylation of C in CPG(s) of the polynucleotide.

In another preferable embodiment, the methylation profile detecting method is not for the purpose of directly obtaining the diagnosis result of a disease, or is not a diagnostic method.

The ninth aspect of the disclosure provides a tumor diagnosis kit, including primer pairs designed based on the sequence described in the first or second aspect of the disclosure, and gene Panels or gene groups containing the sequence, to obtain the characteristics of normal cells and tumor cells through DNA methylation detection.

Other aspects of the disclosure will be apparent to those skilled in the art based on the disclosure herein.

### BRIEF DESCRIPTION OF DRAWINGS

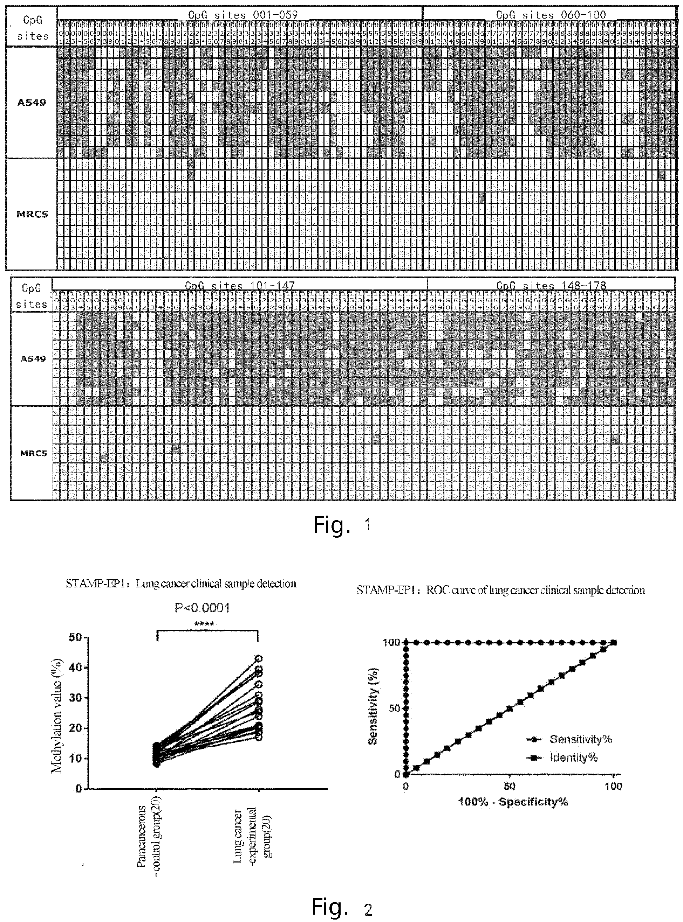

Figure 1 shows the average methylation value of STAMP-EP1 in the genomic DNA of lung cancer cell line A549, with the corresponding value of the genomic DNA of normal lung fibroblast cell line MRC5 as the control. Dark squares indicate the corresponding sites "methylated", while light squares indicate the corresponding sites "non-methylated".
Figure 2 shows comparison of STAMP-EP1 methylation value (left), the detection specificity and sensitivity (right) between control group and experimental group of 20 pairs of paracancerous-lung cancer samples, with paracancerous samples as lung cancer control group, and lung cancer samples as lung cancer experimental group.

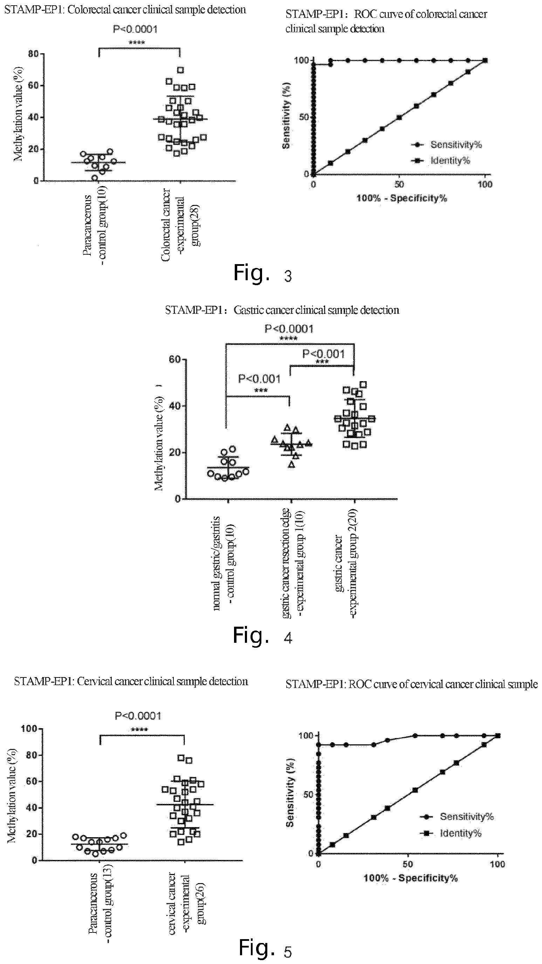

Figure 3. 10 paracancerous clinical samples of colorectal cancer were used as the control group, and 28 colorectal cancer clinical samples were used as the experimental group. The STAMP-EP1 methylation value (left), the detection specificity and sensitivity (right) were compared between the control group and the experimental group.
Figure 4. 10 normal gastric (or gastritis) clinical samples were used as the control group, 10 gastric cancer resection edge clinical samples were used as the experimental group 1, and 20 gastric cancer clinical samples were used as experimental group 2. The STAMP-EP1 methylation value was compared among the three groups.
Figure 5. 13 paracancerous clinical samples of cervical cancer were used as the control group, and 26 cervical cancer clinical samples were used as the experimental group. The STAMP-EP1 methylation value (left), the detection specificity and sensitivity (right) were compared between the control group and the experimental group.

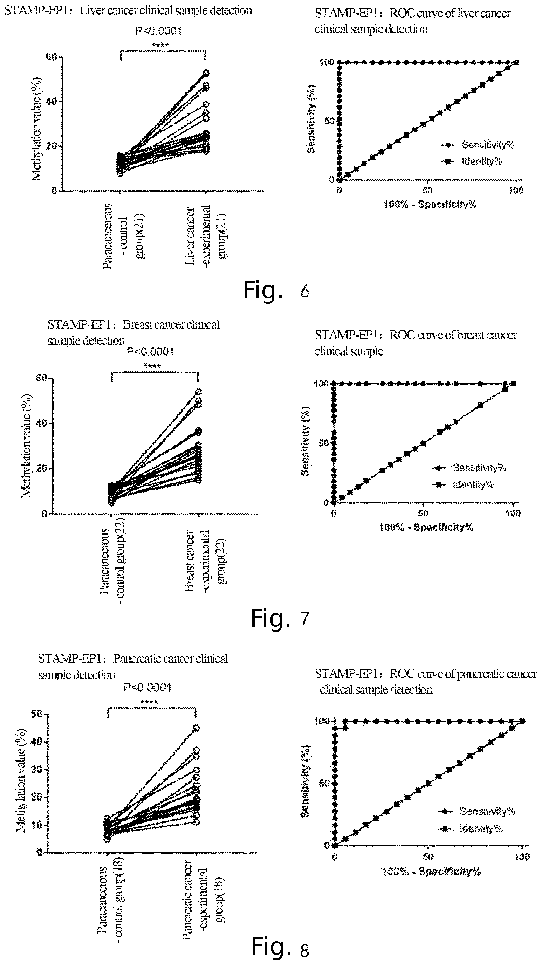

Figure 6 shows comparison of STAMP-EP1 methylation value (left), the detection specificity and sensitivity (right) between control group and experimental group of 21 pairs of paracancerous-liver cancer samples, with paracancerous samples as control group, and liver cancer samples as experimental group.
Figure 7 shows comparison of STAMP-EP1 methylation value (left), the detection specificity and sensitivity (right) between control group and experimental group of 22 pairs of paracancerous-breast cancer samples, with paracancerous samples as control group, and breast cancer samples as experimental group.
Figure 8 shows comparison of STAMP-EP1 methylation value (left), the detection specificity and sensitivity (right) between control group and experimental group of 18 pairs of paracancerous-pancreatic cancer samples, with paracancerous samples as control group, and pancreatic cancer samples as experimental group.

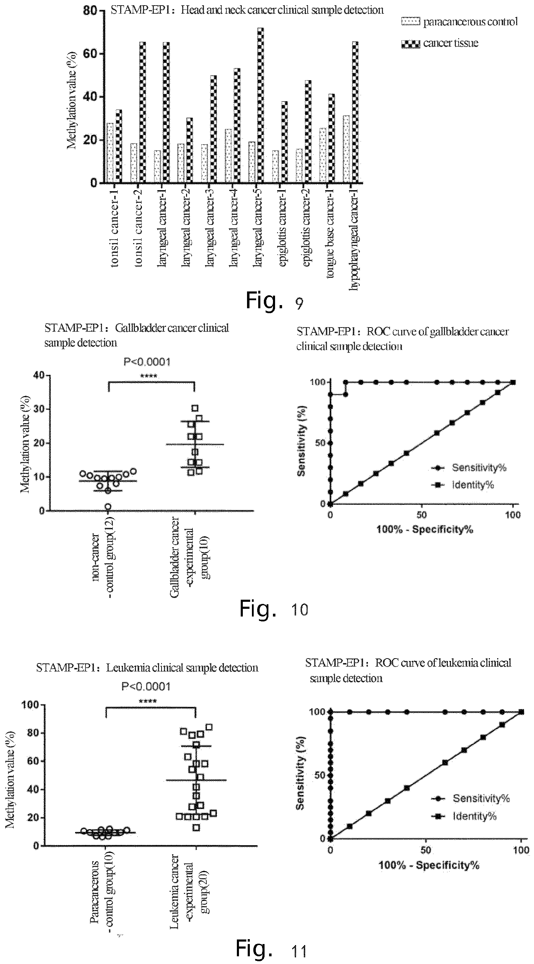

Figure 9. Eleven pairs of paracancerous-head and neck cancer samples were obtained, including 5 cases of laryngeal cancer, 2 cases of tonsil cancer, 2 cases of epiglottis cancer, 1 case of tongue base cancer, and 1 case of hypopharyngeal cancer. STAMP-EP1 methylation value was compared between head and neck cancer control group and experimental group, with paracancerous samples as control group, and cancer samples as experimental group.
Figure 10 shows comparison of STAMP-EP1 methylation value (left), the detection specificity and sensitivity (right) between control group and experimental group of bile samples from 12 non-cancer patients and 10 gallbladder cancer patients, with non-cancer samples as control group, and gallbladder cancer samples as experimental group.
Figure 11. Ten non-leukemia bone marrow smear samples were used as the control group, and 20 leukemia bone marrow smear samples were used as the experimental group. The STAMP-EP1 methylation value (left), the detection specificity and sensitivity (right) were compared between the control group and experimental group.

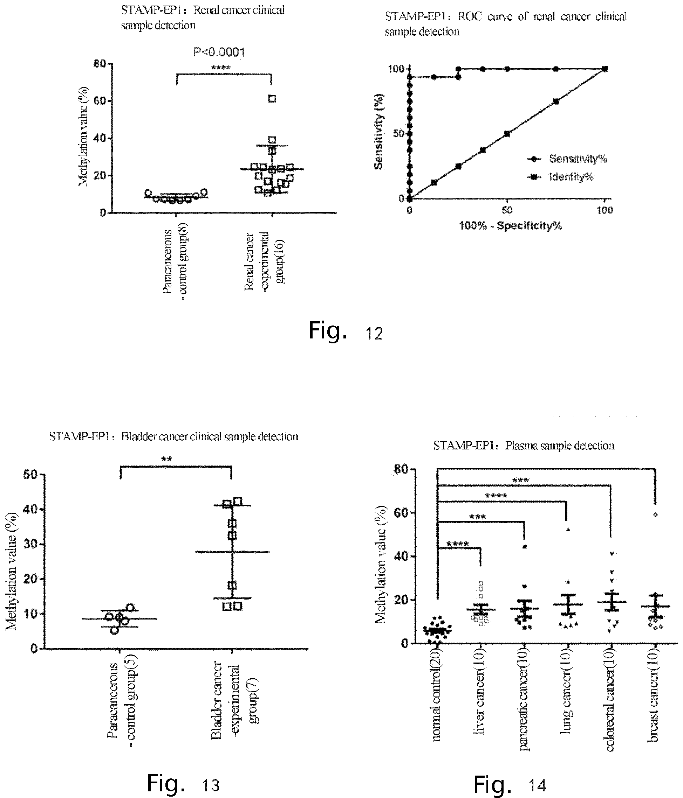

Figure 12. Eight paracancerous clinical samples of renal cancer were used as the control group, and 16 renal cancer clinical samples were used as the experimental group. The STAMP-EP1 methylation value (left), the detection specificity and sensitivity (right) were compared between the control group and experimental group.
Figure 13. Five cases of bladder cancer paracancerous samples and non-cancer urine samples were used as the control group, and 7 cases of bladder cancer tissue samples and bladder cancer urine samples were used as the experimental group. The STAMP-EP1 methylation value was compared between the control group and the experimental group.
Figure 14. Twenty normal human plasma samples were used as the control group, and plasma samples from patients with different tumor types were obtained, including 10 cases of liver cancer, 10 cases of pancreatic cancer, 10 cases of lung cancer, 10 cases of colorectal cancer and 10 cases of breast cancer. The STAMP-EP1 methylation value was compared between the control group and the experimental group.

### DETAILED DESCRIPTION

The inventor is committed to the research of tumor markers. After extensive research and screening, the inventor provides a universal DNA methylation tumor marker, STAMP (Specific Tumor Aligned Methylation of Pan-cancer). In normal tissues, STAMP was hypomethylated, while in tumor tissues, it was hypermethylated. It can be used for clinical tumor detection and as the basis of designing tumor diagnostic agents.

### Term

As used herein, "isolated" refers to a material separated from its original environment (if the material is a natural material, the original environment is the natural environment). For example, in living cells, polynucleotides and polypeptides in their natural state are not isolated or purified, but the same polynucleotides or polypeptides will be isolated ones if they are separated from other substances existed in the natural state.

As used herein, "sample" includes substances suitable for DNA methylation detection obtained from any individual or isolated tissue, cell or body fluid (such as plasma). For example, the samples include but are not limited to: tissue samples, paraffin embedded samples, blood samples, pleural effusion samples, and alveolar lavage fluid samples, ascites and lavage fluid samples, bile samples, stool samples, urine samples, saliva samples, cerebrospinal fluid samples, cell smear samples, cervical scraping or brushing samples, tissue and cell biopsy samples.

As used herein, "hypermethylation" refers to high level of methylation, hydroxymethylation, aldehyde methylation or carboxymethylation of CpG in a gene sequence. For example, in the case of methylation specific PCR (MSP), if the PCR reaction with methylation specific primers has positive PCR results, the DNA (gene) region of interest is in hypermethylation state. For another example, in the case of real-time quantitative methylation specific PCR, hypermethylation can be determined based on statistic difference of the methylation status value as compared with the control sample.

As used herein, the tumors include but are not limited to: hematologic cancers such as leukemia, lymphoma, multiple myeloma; digestive system tumors such as esophageal cancer, gastric cancer, colorectal cancer, liver cancer, pancreatic cancer, bile duct and gallbladder cancer; respiratory system tumors such as lung cancer, pleuroma; nervous system tumors such as glioma, neuroblastoma, meningioma; head and neck tumors such as oral cancer, tongue cancer, laryngeal cancer, nasopharyngeal cancer; gynecological and reproductive system tumors such as breast cancer, ovarian cancer, cervical cancer, vulvar cancer, testicular cancer, prostate cancer, penile cancer; urinary system tumors such as kidney cancer, bladder cancer, skin and other systems tumors such as skin cancer, melanoma, osteosarcoma, liposarcoma, thyroid cancer.

### Gene marker

In order to find a useful target for tumor diagnosis, the inventor has identified the target of STAMP-EP1 after extensive and in-depth research. The methylation status of the sequence of STAMP-EP1 gene is significantly different between tumor tissues and non-tumor tissues. If the abnormal hypermethylation of the sequence of STAMP-EP1 gene of a subject is detected, the subject can be identified as having a high-risk of tumor. Moreover, the significant difference of STAMP-EP1 between tumor and non-tumor tissues exists in different types of tumors, including solid tumors and non-solid tumors.

Therefore, the disclosure provides an isolated polynucleotide, comprising the nucleotide sequence shown in the sequence of SEQ ID NO: 1 or SEQ ID NO: 3 (the reverse complementary sequence of SEQ ID NO: 1). For tumor cells, the polynucleotide contains 5-methylcytosine (5mC) at C positions of many 5'-CpG-3'. The disclosure also comprises fragments of the polynucleotide of the sequence shown in SEQ ID NO: 1 or 3, having at least one (such as 2-178, more specifically 3, 5, 10, 15, 20, 25, 30, 50, 60, 80, 100, 120, 150) methylated CpG site. The above polynucleotides or fragments can also be used in the design of detection agents or detection kits.

In some specific embodiments of the disclosure, the fragments of the polynucleotide are, for example, a fragment containing the residues 1-589 of SEQ ID NO: 1 (containing CpG sites 001-059); a fragment containing the residues 632-1218 of SEQ ID NO: 1 (containing CpG sites 060-100); a fragment containing the residues 1322-2066 of SEQ ID NO: 1 (containing CpG sites 101-147); a fragment containing the residues 2100-2448 of SEQ ID NO: 1 (containing CpG sites 148-178). Antisense chains of the above fragments are suitable for use in the disclosure. These fragments are merely examples of preferable embodiments of the present disclosure. Based on the information provided by the present disclosure, other fragments can also be selected.

In addition, gene Panels or gene groups containing the sequence shown in the SEQ ID NO: 1 or SEQ ID NO: 2 or fragments thereof are also encompassed by the disclosure. For the gene Panel or gene group, the characteristics of normal cells and tumor cells can also be identified through DNA methylation detection.

The above polynucleotides can be used as the key regions for analysis of the methylation status in the genome. Their methylation status can be analyzed by various technologies known in the art. Any technique that can be used to analyze the methylation state can be applied to the present disclosure.

When treated with bisulfite, un-methylated cytosine(s) of the above polynucleotides will be converted into uracil, while methylated cytosine(s) remained unchanged.

Therefore, the disclosure also provides the polynucleotides obtained from the above polynucleotides (including the complementary chain (antisense chain)) after being treated with bisulfite, including the polynucleotides of the sequence shown in SEQ ID NO: 2 or SEQ ID NO: 4. These polynucleotides can also be used in the design of detection agents or detection kits.

The disclosure also comprises fragments of the polynucleotides obtained from the above polynucleotides or the antisense chain thereof after being treated with bisulfite, wherein the fragments contain at least one methylated CpG site.

### Detection agents and kits

Based on the new discovery of the disclosure, a detection agent designed based on said polynucleotide(s) is also provided for detecting the methylation profile of polynucleotide(s) in the sample *in vitro.* The detection methods and agents known in the art for determining the sequence, variation and methylation of the genome can be applied in the disclosure.

Therefore, the disclosure provides a method of preparing a tumor detection agent, including: providing the polynucleotide, designing a detection agent for specifically detecting a target sequence which is the full length or fragment of the polynucleotide; wherein, the target sequence has at least one methylated CpG site.

The detection agent herein includes but is not limited to: primers, probes, etc.

For example, the agent is primer pairs. Based on the sequence of the polynucleotide, those skilled in the art know how to design primer(s). The two primers are on each side of the specific sequence of the target gene to be amplified (including CpG sequence, for the gene region originally methylated, the primer is complementary with CpG, and for the gene region originally un-methylated, the primer is complementary with TpG). It should be understood that based on the new discovery of the disclosure, those skilled in the art can design a variety of primers or probes or other types of detection agents for CpG sites at different positions on the target sequence or their combinations. These primers or probes or other types of detection agents should be included in the technical solution of the present disclosure.

The agent can also be a combination of agents (primer combination), including more than one set of primers, so that the multiple polynucleotides can be amplified respectively.

The disclosure also provides a kit for detecting the methylation profile of polynucleotide in a sample *in vitro,* which comprises container(s) and the above primer pair(s) in the container(s).

In addition, the kit can also include various reagents required for DNA extraction, DNA purification, PCR amplification, etc.

In addition, the kit can also include an instruction for use, which indicates operation steps of the detection and a result judgment standard, for the application of those skilled in the art.

### Detection method

The methylation profile of a polynucleotide can be determined by any technique in the art (such as methylation specific PCR (MSP) or real-time quantitative methylation specific PCR, Methylight), or other techniques that are still developing and will be developed.

Quantitative methylation specific PCR (QMSP) can also be used to detect methylation level. It is a continuous optical monitoring method based on fluorescent PCR, which is more sensitive than MSP. It has high throughput and avoids electrophoresis based result analysis.

Other available technologies include conventional methods in the art such as pyrosequencing, bisulfite converstion sequencing, qPCR, second generation sequencing, whole genome methylation sequencing, DNA enrichment detection, simplified bisulfite sequencing or HPLC, and combined gene group detection. It should be understood that, on the basis of the new disclosure herein, these well-known technologies and some technologies to be developed in the art can be applied to the present disclosure.

As a preferable embodiment of the disclosure, a method of detecting the methylation profile of a polynucleotide in a sample *in vitro* is also provided. The method is based on the follow principle: the un-methylated cytosine can be converted into uracil by bisulfite, which can be transformed into thymine in the subsequent PCR amplification process, while the methylated cytosine remains unchanged; therefore, after the polynucleotide is treated by bisulfite, the methylated site presents a polynucleotide polymorphism (SNP) similar to C/T. Based on the above principle, methylated and un-methylated cytosine can be distinguished effectively by identifying the methylation profile of a polynucleotide in the sample.

The method of the disclosure includes: (a) providing samples and extracting genomic DNA; (b) treating the genomic DNA of step (a) with bisulfite, so as to convert the un-methylated cytosine in the genomic DNA into uracil; (c) analyzing whether the genomic DNA treated in step (b) contains an abnormal methylation profile.

The method of the disclosure can be used for: (i) analyzing whether a subject has tumor by detecting the sample of the subject; (ii) identifying a population having high-risk of tumor. The method needs not to be aimed at obtaining direct diagnosis results.

In a preferable embodiment of the disclosure, DNA methylation is detected by PCR amplification and pyrosequencing. It should be understood by those in the art that DNA methylation detection is not limited to these methods, and any other DNA methylation detection method can also be used. The primers used in PCR amplification are not limited to those provided in Examples.

Because of bisulfite treatment, in which un-methylated cytosine in genomic DNA are converted into uracil and then transformed into thymine in the subsequent PCR process, the sequence complexity of the genome will be reduced, and it will be more difficult to amplify specific target fragments by PCR. Therefore, in order to improve amplification efficiency and specificity, nested PCR may be preferable, wherein two sets of primers (outer primers and inner primers) are used in two successive runs of PCR, and the amplification product from the first run undergoes a second run with the second set of primers. However, it should be understood that the detection methods suitable for the present disclosure are not limited thereto.

After the research and verification on clinical samples, the method of the disclosure provides very high accuracy in the clinical diagnosis of tumors. The disclosure can be applied to the fields of tumor auxiliary diagnosis, efficacy evaluation, prognosis monitoring, etc., thus has a high clinical value.

The disclosure is further illustrated by the specific examples described below. It should be understood that these examples are merely illustrative, and do not limit the scope of the present disclosure. The experimental methods without specifying the specific conditions in the following examples generally used the conventional conditions, such as those described in J. Sambrook, Molecular Cloning: A Laboratory Manual (3rd ed. Science Press, 2002) or followed the manufacturer's recommendation.

### Example 1. Nucleic acid sequence for STAMP-EP1 detection

The sequence of the STAMP-EP1 tumor marker is provided as follows: SEQ ID NO: 1 (chr14: 60975733bp ∼ 60978180bp (hg19/Human)), in which the underlined bases are methylated CpG sites, and each number below the underline indicates the site number.

1~50bp CGGCGGGCGC TGTCGAGCAC GGGGAGGTGC TGAAATAGTC CTGGCGTGCT
001 002 003 004 005 006
51~100bp GATTCAAGCT TTGATTGGCA GAGCCACCCG GTGACTGACA GGGGGTCTCC
007
101~150bp ATGGCGCCCG CGCCGCCAAT CCGCCCACCC CAATAGCGGA GCCAGCTCGC
008 009 010 011 012 013 014
151~200bp CTGCCGGCGT GCCTGAGCCG AGCCGAGCCC GAACCCCAAG CCGCGGAGCC
015 016 017 018 019 020 021
201~250bp AGCACCTCCT CCAGTCGGGG TCGTCCGCTC CCGGCCGTTG AGCCACCGCC
022 023 024 025 026 027 028
251~300bp GCCACCCGGT AGTGTGTCCC GCTGCCCCAA TCCGCCTCAT CAACAAGCGC
029 030 031 032
301~350bp CTGGCACACT CAGCCAGGCC CGCGGGCATC TGCTGCGTGT CCCGCTCCGG
033 034 035 036 037
351~400bp GCTCAGTGCC CTCGCCGCCG CCGGCACTGC CTCGATGTTC CAGCTGCCCA
038 039 040 041 042
401~450bp TCTTGAATTT CAGCCCCCAG CAAGTGGCCG GGGTATGTGA GACCCTGGAA
043
451~500bp GAGAGCGGCG ATGTGGAGCG CCTGGGTCGC TTCCTCTGGT CGCTGCCCGT
044 045 046 047 048 049
501~550bp GGCCCCTGCG GCCTGCGAGG CCCTCAACAA GAATGAGTCG GTGCTACGCG
050 051 052 053 054
551~600bp CACGAGCCAT CGTGGCCTTT CACGGTGGCA ACTACCGCGA GCTCTATCAT
055 056 057 058 059
601~650bp ATCCTGGAAA ACCACAAGTT CACCAAGGAG TCGCACGCCA AGCTGCAGGC
060 061 062
651~700bp GCTGTGGCTT GAAGCACACT ACCAGGAGGC TGAGAAGCTG CGTGGAAGAC
063
701~750bp CCCTGGGACC TGTGGACAAG TACCGAGTAA GGAAGAAGTT CCCGCTGCCG
064 065 066
751~800bp CGCACCATTT GGGACGGCGA ACAGAAGACA CACTGCTTCA AGGAGCGCAC
067 068 069 070 071
801~850bp GCGGCACCTG CTACGCGAGT GGTACCTGCA GGATCCATAC CCTAACCCCA
072 073 074
851~900bp GCAAAAAACG TGAGCTCGCC CAGGCAACCG GACTGACCCC TACGCAGGTG
075 076 077 078
901~950bp GGCAACTGGT TCAAAAACCG CCGACAAAGG GACCGAGCGG CTGCAGCCAA
079 080 081 082
951~1000bp GAACAGGTCG GTACCTAGAG GCCTCCGCGC TTTGAGCGCA CCGGGGAGGA
083 084 085 086 087
1001~1050bp GGCGGGTGGA GGCACCTCTG GCGCCCTTAC CCAGTCCCTG GCGACTCCAA
088 089 090
1051~1100bp TTCAGCAGGA GTTGGGAGCG CGGTCTGTCT TGGGTTAAGA GCCCTGCGTT
091 092 093
1101~1150bp CTGGGCTCCT GGCCGGGAGT TCCCTTGCCG GCTCTGCTTC CCCACCCGCT
094 095 096
1151~1200bp GGCTCCCCAC GCCTGCGGGC AGCTGCAGCA GCTGGTCCCG GTCACCAAAC
097 098 099
1201~1250bp CAAGGCTTCA CTGGGACGGA GAGGGGAAGA GAAATAAAAA ATTAAAATCC
100
1251~1300bp TACAAACAGT TAGGGACCCC AAGACCCAAA GCTAATTCTT GTCAGCCTGG
1301~1350bp GCACAGGCTC CTACTATTAA TCGAAGCCTG GCTTATTAGC AATGTGTCGG
101 102
1351~1400bp TTTCATGTTA ATTATCATTT TCAAAGCCCA GGTATATCCC TCCCTAATGC
1401~1450bp TTTGAAAACA GTTTTCAATG GACTTTTGAG AAATGGGAAG TCGAGTTTTC
103
1451~1500bp CTCTTCCCAT GCGCTGCCTG CCACTCTTGT CTCAAAACAG CAAACTAGTC
104
1501~1550bp CGTGGGCCGA GGCTTTTCGT TTCCCGGAGT GTGGATCTCG ATTAGCCAAA
105 106 107 108 109
1551~1600bp CATTTTGCGG AAGAGCCCGG CCTCATCCCC CAGGCCCAAA TGCTCCTTAC
110 111
1601~1650bp AATCCTTTTT GCCTTTAGGT CGGGCCGACC CGATCCAACG CGATCGCGGG
112 113 114 115 116 117 118
1651~1700bp AGCACTTGCT CAGGCGTAAG CCCCAGGCAG ACGCACCGTT AGAAATGGTA
119 120 121
1701~1750bp TCCCATGTCC CTGGGACCGA TCTGTCCTTG TCACCCACAC TTCGTTTATT
122 123
1751~1800bp TCCTGACAGT CCTGTAAATC TCCCAAAAGT GCACAACAAA CAGGGAGGAC
1801~1850bp ACTGCAAGCC CAGTATATAA AAGACCTGGG AGCTGCGGCG CTGAGAAAGG
124 125
1851~1900bp GCGCGAATCA TGGTGGGGCA CAACAGTAGG GACCCGCGGA GGGGCGGCCG
126 127 128 129 130 131

1901~1950bp CGGACTCCTG CCCGACCTCT GTCGCCTTGC CGAGTAATCC TCGCCTTAAC
132 133 134 135 136
1951~2000bp TGCTGGGGTC TTCGGAAGAA CCTCTAGCCG CCGGGCTGGA GGGACGCAGG
137 138 139 140
2001~2050bp AGGTGGTGGG GGCGGGCGAC GGGCGGCTGT GTTACGAGCT GTGACCCGTG
141 142 143 144 145 146
2051~2100bp TTCCCTTTCT TCCCCGTAGA CTCCAGCAGC AGGTCCTGTC ACAGGGTTCC
147 148
2101~2150bp GGGCGGGCAC TACGGGCGGA GGGCGACGGC ACGCCAGAGG TGCTGGGCGT
149 150 151 152 153 154 155
2151~2200bp CGCCACCAGC CCGGCCGCCA GTCTATCCAG CAAGGCGGCC ACTTCAGCCA
156 157 158 159
2201~2250bp TCTCCATCAC GTCCAGCGAC AGCGAGTGCG ACATCTGAGT TGCCCATCCA
160 161 162 163
2251~2300bp GGATGCTCAG AAGCAGATTC CAGTGTAAAA ACGAGAAAAA CAAAATGAAA
164
2301~2350bp GAGGGGAAGA AGATGAGAGA CCTGCAAATC CAGCGCCACA GAAGCCAGGT
165
2351~2400bp GACCAGGGAC CCGCGGGCTC GGGTTGCCGT TTCCCGCCCC ACCCCGCGGC
166 167 168 169 170 171 172
2401~2448bp CGGCCTGGCT TCACTGGCGC CCTTTGGCCG CGACCACGGG AACCAGCG
173 174 175 176 177 178

The bisulfite treated sequence from SEQ ID NO: 1 is shown in SEQ ID NO: 2 (Y represents C or U) as follows:

1~50bp YGGYGGGYGU TGTYGAGUAY GGGGAGGTGU TGAAATAGTU UTGGYGTGUT
001 002 003 004 005 006
51~100bp GATTUAAGUT TTGATTGGUA GAGUUAUUYG GTGAUTGAUA GGGGGTUTUU
007
101~150bp ATGGYGUUYG YGUYGUUAAT UYGUUUAUUU UAATAGYGGA GUUAGUTYGU
008 009 010 011 012 013 014
151~200bp UTGUYGGYGT GUUTGAGUYG AGUYGAGUUY GAAUUUUAAG UYGYGGAGUU
015 016 017 018 019 020 021
201~250bp AGUAUUTUUT UUAGTYGGGG TYGTUYGUTU UYGGUYGTTG AGUUAUYGUY
022 023 024 025 026 027 028
251~300bp GUUAUUYGGT AGTGTGTUUY GUTGUUUUAA TUYGUUTUAT UAAUAAGYGU
029 030 031 032
301~350bp UTGGUAUAUT UAGUUAGGUU YGYGGGUATU TGUTGYGTGT UUYGUTUYGG
033 034 035 036 037
351~400bp GUTUAGTGUU UTYGUYGUYG UYGGUAUTGU UTYGATGTTU UAGUTGUUUA
038 039 040 041 042
401~450bp TUTTGAATTT UAGUUUUUAG UAAGTGGUYG GGGTATGTGA GAUUUTGGAA
043
451~500bp GAGAGYGGYG ATGTGGAGYG UUTGGGTYGU TTUUTUTGGT YGUTGUUYGT
044 045 046 047 048 049
501~550bp GGUUUUTGYG GUUTGYGAGG UUUTUAAUAA GAATGAGTYG GTGUTAYGYG
050 051 052 053 054
551~600bp UAYGAGUUAT YGTGGUUTTT UAYGGTGGUA AUTAUYGYGA GUTUTATUAT
055 056 057 058 059
601~650bp ATUUTGGAAA AUUAUAAGTT UAUUAAGGAG TYGUAYGUUA AGUTGUAGGY
060 061 062
651~700bp GUTGTGGUTT GAAGUAUAUT AUUAGGAGGU TGAGAAGUTG YGTGGAAGAU
063
701~750bp UUUTGGGAUU TGTGGAUAAG TAUYGAGTAA GGAAGAAGTT UUYGUTGUYG
064 065 066
751~800bp YGUAUUATTT GGGAYGGYGA AUAGAAGAUA UAUTGUTTUA AGGAGYGUAY
067 068 069 070 071
801~850bp GYGGUAUUTG UTAYGYGAGT GGTAUUTGUA GGATUUATAU UUTAAUUUUA
072 073 074
851~900bp GUAAAAAAYG TGAGUTYGUU UAGGUAAUYG GAUTGAUUUU TAYGUAGGTG
075 076 077 078
901~950bp GGUAAUTGGT TUAAAAAUYG UYGAUAAAGG GAUYGAGYGG UTGUAGUUAA
079 080 081 082
951~1000bp GAAUAGGTYG GTAUUTAGAG GUUTUYGYGU TTTGAGYGUA UYGGGGAGGA
083 084 085 086 087
1001~1050bp GGYGGGTGGA GGUAUUTUTG GYGUUUTTAU UUAGTUUUTG GYGAUTUUAA
088 089 090
1051~1100bp TTUAGUAGGA GTTGGGAGYG YGGTUTGTUT TGGGTTAAGA GUUUTGYGTT
091 092 093
1101~1150bp UTGGGUTUUT GGUYGGGAGT TUUUTTGUYG GUTUTGUTTU UUUAUUYGUT
094 095 096
1151~1200bp GGUTUUUUAY GUUTGYGGGU AGUTGUAGUA GUTGGTUUYG GTUAUUAAAU
097 098 099

1201~1250bp UAAGGUTTUA UTGGGAYGGA GAGGGGAAGA GAAATAAAAA ATTAAAATUU
100
1251~1300bp TAUAAAUAGT TAGGGAUUUU AAGAUUUAAA GUTAATTUTT GTUAGUUTGG
1301~1350bp GUAUAGGUTU UTAUTATTAA TYGAAGUUTG GUTTATTAGU AATGTGTYGG
101 102
1351~1400bp TTTUATGTTA ATTATUATTT TUAAAGUUUA GGTATATUUU TUUUTAATGU
1401~1450bp TTTGAAAAUA GTTTTUAATG GAUTTTTGAG AAATGGGAAG TYGAGTTTTU
103
1451~1500bp UTUTTUUUAT GYGUTGUUTG UUAUTUTTGT UTUAAAAUAG UAAAUTAGTU
104
1501~1550bp YGTGGGUYGA GGUTTTTYGT TTUUYGGAGT GTGGATUTYG ATTAGUUAAA
105 106 107 108 109
1551~1600bp UATTTTGYGG AAGAGUUYGG UUTUATUUUU UAGGUUUAAA TGUTUUTTAU
110 111
1601~1650bp AATUUTTTTT GUUTTTAGGT YGGGUYGAUU YGATUUAAYG YGATYGYGGG
112 113 114 115 116 117 118
1651~1700bp AGUAUTTGUT UAGGYGTAAG UUUUAGGUAG AYGUAUYGTT AGAAATGGTA
119 120 121
1701~1750bp TUUUATGTUU UTGGGAUYGA TUTGTUUTTG TUAUUUAUAU TTYGTTTATT
122 123
1751~1800bp TUUTGAUAGT UUTGTAAATU TUUUAAAAGT GUAUAAUAAA UAGGGAGGAU
1801~1850bp AUTGUAAGUU UAGTATATAA AAGAUUTGGG AGUTGYGGYG UTGAGAAAGG
124 125
1851~1900bp GYGYGAATUA TGGTGGGGUA UAAUAGTAGG GAUUYGYGGA GGGGYGGUYG
126 127 128 129 130 131
1901~1950bp YGGAUTUUTG UUYGAUUTUT GTYGUUTTGU YGAGTAATUU TYGUUTTAAU
132 133 134 135 136
1951~2000bp TGUTGGGGTU TTYGGAAGAA UUTUTAGUYG UYGGGUTGGA GGGAYGUAGG
137 138 139 140
2001~2050bp AGGTGGTGGG GGYGGGYGAY GGGYGGUTGT GTTAYGAGUT GTGAUUYGTG
141 142 143 144 145 146
2051~2100bp TTUUUTTTUT TUUUYGTAGA UTUUAGUAGU AGGTUUTGTU AUAGGGTTUY
147 148
2101~2150bp GGGYGGGUAU TAYGGGYGGA GGGYGAYGGU AYGUUAGAGG TGUTGGGYGT
149 150 151 152 153 154 155
2151~2200bp YGUUAUUAGU UYGGUYGUUA GTUTATUUAG UAAGGYGGUU AUTTUAGUUA
156 157 158 159
2201~2250bp TUTUUATUAY GTUUAGYGAU AGYGAGTGYG AUATUTGAGT TGUUUATUUA
160 161 162 163
2251~2300bp GGATGUTUAG AAGUAGATTU UAGTGTAAAA AYGAGAAAAA UAAAATGAAA
164
2301~2350bp GAGGGGAAGA AGATGAGAGA UUTGUAAATU UAGYGUUAUA GAAGUUAGGT
165
2351~2400bp GAUUAGGGAU UYGYGGGUTY GGGTTGUYGT TTUUYGUUUU AUUUYGYGGU
166 167 168 169 170 171 172
2401~2448bp YGGUUTGGUT TUAUTGGYGU UUTTTGGUYG YGAUUAYGGG AAUUAGYG
173 174 175 176 177 178

The reverse complementary sequence of SEQ ID NO: 1 is shown in SEQ ID NO: 3 as follows:

CGCTGGTTCCCGTGGTCGCGGCCAAAGGGCGCCAGTGAAGCCAGGCCGGCCGCGGGGTGGGGCGGAAACGGCAACCCGAGCCCGCGGGTCCCTGGT
CACCTGGCTTCTGTGGCGCTGGATTTGCAGGTCTCTCATCTTCTTCCCCTCTTTCATTTTGTTTTTCTCGTTTTTACACTGGAATCTGCTTCTGAGC
ATCCTGGATGGGCAACTCAGATGTCGCACTCGCTGTCGCTGGACGTGATGGAGATGGCTGAAGTGGCCGCCTTGCTGGATAGACTGGCGGCCGGGCT
GGTGGCGACGCCCAGCACCTCTGGCGTGCCGTCGCCCTCCGCCCGTAGTGCCCGCCCGGAACCCTGTGACAGGACCTGCTGCTGGAGTCTACGGGGA
AGAAAGGGAACACGGGTCACAGCTCGTAACACAGCCGCCCGTCGCCCGCCCCCACCACCTCCTGCGTCCCTCCAGCCCGGCGGCTAGAGGTTCTTCC
GAAGACCCCAGCAGTTAAGGCGAGGATTACTCGGCAAGGCGACAGAGGTCGGGCAGGAGTCCGCGGCCGCCCCTCCGCGGGTCCCTACTGTTGTGCC
CCACCATGATTCGCGCCCTTTCTCAGCGCCGCAGCTCCCAGGTCTTTTATATACTGGGCTTGCAGTGTCCTCCCTGTTTGTTGTGCACTTTTGGGAG
ATTTACAGGACTGTCAGGAAATAAACGAAGTGTGGGTGACAAGGACAGATCGGTCCCAGGGACATGGGATACCATTTCTAACGGTGCGTCTGCCTGG
GGCTTACGCCTGAGCAAGTGCTCCCGCGATCGCGTTGGATCGGGTCGGCCCGACCTAAAGGCAAAAAGGATTGTAAGGAGCATTTGGGCCTGGGGGA
TGAGGCCGGGCTCTTCCGCAAAATGTTTGGCTAATCGAGATCCACACTCCGGGAAACGAAAAGCCTCGGCCCACGGACTAGTTTGCTGTTTTGAGAC
AAGAGTGGCAGGCAGCGCATGGGAAGAGGAAAACTCGACTTCCCATTTCTCAAAAGTCCATTGAAAACTGTTTTCAAAGCATTAGGGAGGGATATAC
CTGGGCTTTGAAAATGATAATTAACATGAAACCGACACATTGCTAATAAGCCAGGCTTCGATTAATAGTAGGAGCCTGTGCCCAGGCTGACAAGAAT
TAGCTTTGGGTCTTGGGGTCCCTAACTGTTTGTAGGATTTTAATTTTTTATTTCTCTTCCCCTCTCCGTCCCAGTGAAGCCTTGGTTTGGTGACCGG
GACCAGCTGCTGCAGCTGCCCGCAGGCGTGGGGAGCCAGCGGGTGGGGAAGCAGAGCCGGCAAGGGAACTCCCGGCCAGGAGCCCAGAACGCAGGGC
TCTTAACCCAAGACAGACCGCGCTCCCAACTCCTGCTGAATTGGAGTCGCCAGGGACTGGGTAAGGGCGCCAGAGGTGCCTCCACCCGCCTCCTCCC
CGGTGCGCTCAAAGCGCGGAGGCCTCTAGGTACCGACCTGTTCTTGGCTGCAGCCGCTCGGTCCCTTTGTCGGCGGTTTTTGAACCAGTTGCCCACC
TGCGTAGGGGTCAGTCCGGTTGCCTGGGCGAGCTCACGTTTTTTGCTGGGGTTAGGGTATGGATCCTGCAGGTACCACTCGCGTAGCAGGTGCCGCG
TGCGCTCCTTGAAGCAGTGTGTCTTCTGTTCGCCGTCCCAAATGGTGCGCGGCAGCGGGAACTTCTTCCTTACTCGGTACTTGTCCACAGGTCCCAG
GGGTCTTCCACGCAGCTTCTCAGCCTCCTGGTAGTGTGCTTCAAGCCACAGCGCCTGCAGCTTGGCGTGCGACTCCTTGGTGAACTTGTGGTTTTCC
AGGATATGATAGAGCTCGCGGTAGTTGCCACCGTGAAAGGCCACGATGGCTCGTGCGCGTAGCACCGACTCATTCTTGTTGAGGGCCTCGCAGGCCG

CAGGGGCCACGGGCAGCGACCAGAGGAAGCGACCCAGGCGCTCCACATCGCCGCTCTCTTCCAGGGTCTCACATACCCCGGCCACTTGCTGGGGGCT
GAAATTCAAGATGGGCAGCTGGAACATCGAGGCAGTGCCGGCGGCGGCGAGGGCACTGAGCCCGGAGCGGGACACGCAGCAGATGCCCGCGGGCCTG
GCTGAGTGTGCCAGGCGCTTGTTGATGAGGCGGATTGGGGCAGCGGGACACACTACCGGGTGGCGGCGGTGGCTCAACGGCCGGGAGCGGACGACCC
CGACTGGAGGAGGTGCTGGCTCCGCGGCTTGGGGTTCGGGCTCGGCTCGGCTCAGGCACGCCGGCAGGCGAGCTGGCTCCGCTATTGGGGTGGGCGG
ATTGGCGGCGCGGGCGCCATGGAGACCCCCTGTCAGTCACCGGGTGGCTCTGCCAATCAAAGCTTGAATCAGCACGCCAGGACTATTTCAGCACCTC
CCCGTGCTCGACAGCGCCCGCCG

The bisulfite treated sequence from SEQ ID NO: 3 is shown in SEQ ID NO: 4 (Y represents C or U) as follows:

YGUTGGTTUUYGTGGTYGYGGUUAAAGGGYGUUAGTGAAGUUAGGUYGGUYGYGGGGTGGGGYGGGAAAYGGUAAUUYGAGUUYGYGGGTUUUTGGT
UAUUTGGUTTUTGTGGYGUTGGATTTGUAGGTUTUTUATUTTUTTUUUTUTTTUATTTTGTTTTTUTYGTTTTTAUAUTGGAATUTGUTTUTGAGU
ATUUTGGATGGGUAAUTUAGATGTYGUAUTYGUTGTYGUTGGAYGTGATGGAGATGGUTGAAGTGGUYGUUTTGUTGGATAGAUTGGYGGUYGGGUT
GGTGGYGAYGUUUAGUAUUTUTGGYGTGUYGTYGUUUTUYGUUYGTAGTGUUYGUUYGGAAUUUTGTGAUAGGAUUTGUTGUTGGAGTUTAYGGGGA
AGAAAGGGAAUAYGGGTUAUAGUTYGTAAUAUAGUYGUUYGTYGUUYGUUUUUAUUAUUTUUTGYGTUUUTUUAGUUYGGYGGUTAGAGGTTUTTUY
GAAGAUUUUAGUAGTTAAGGYGAGGATTAUTYGGUAAGGYGAUAGAGGTYGGGUAGGAGTUYGYGGUYGUUUUTUYGYGGGTUUUTAUTGTTGTGUU
UUAUUATGATTYGYGUUUTTTUTUAGYGUYGUAGUTUUUAGGTUTTTTATATAUTGGGUTTGUAGTGTUUTUUUTGTTTGTTGTGUAUTTTTGGGAG
ATTTAUAGGAUTGTUAGGAAATAAAYGAAGTGTGGGTGAUAAGGAUAGATYGGTUUUAGGGAUATGGGATAUUATTTUTAAYGGTGYGTUTGUUTGG
GGUTTAYGUUTGAGUAAGTGUTUUYGYGATYGYGTTGGATYGGGTYGGUUYGAUUTAAAGGUAAAAAGGATTGTAAGGAGUATTTGGGUUTGGGGGA
TGAGGUYGGGUTUTTUYGUAAAATGTTTGGUTAATYGAGATUUAUAUTUYGGGAAAYGAAAAGUUTYGGUUUAYGGAUTAGTTTGUTGTTTTGAGAU
AAGAGTGGUAGGUAGYGUATGGGAAGAGGAAAAUTYGAUTTUUUATTTUTUAAAAGTUUATTGAAAAUTGTTTTUAAAGUATTAGGGAGGGATATAU
UTGGGUTTTGAAAATGATAATTAAUATGAAAUYGAUAUATTGUTAATAAGUUAGGUTTYGATTAATAGTAGGAGUUTGTGUUUAGGUTGAUAAGAAT
TAGUTTTGGGTUTTGGGGTUUUTAAUTGTTTGTAGGATTTTAATTTTTTATTTUTUTTUUUUTUTUYGTUUUAGTGAAGUUTTGGTTTGGTGAUYG
GAUUAGUTGUTGUAGUTGUUYGUAGGYGTGGGGAGUUAGYGGGTGGGGAAGUAGAGUYGGUAAGGGAAUTUUYGGUUAGGAGUUUAGAAYGUAGGGU
TUTTAAUUUAAGAUAGAUYGYGUTUUUAAUTUUTGUTGAATTGGAGTYGUUAGGGAUTGGGTAAGGGYGUUAGAGGTGUUTUUAUUYGUUTUUTUUU
YGGTGYGUTUAAAGYGYGGAGGUUTUTAGGTAUYGAUUTGTUTTGGUTGUAGUYGUTYGGTUUUTTTGTYGGYGGTTTTTGAAUUAGTTGUUUAUU
TGYGTAGGGGTUAGTUYGGTTGUUTGGGYGAGUTUAYGTTTTTTGUTGGGGTTAGGGTATGGATUUTGUAGGTAUUAUTYGYGTAGUAGGTGUYGYG
TGYGUTUUTTGAAGUAGTGTGTUTTUTGTTYGUUYGTUUUAAATGGTGYGYGGUAGYGGGAAUTTUTTUUTTAUTYGGTAUTTGTUUAUAGGTUUUAG
GGGTUTTUUAYGUAGUTTUTUAGUUTUUTGGTAGTGTGUTTUAAGUUAUAGYGUUTGUAGUTTGGYGTGYGAUTUUTTGGTGAAUTTGTGGTTTTUU
AGGATATGATAGAGUTYGYGGTAGTTGUUAUYGTGAAAGGUUAYGATGGUTYGTGYGYGTAGUAUYGAUTUATTUTTGTTGAGGGUUTYGUAGGUYG
UAGGGGUUAYGGGUAGYGAUUAGAGGAAGYGAUUUAGGYGUTUUAUATYGUUYGUTUTUTTUUAGGGTUTUAUATAUUUYGGUUAUTTGUTGGGGGUT
GAAATTUAAGATGGGUAGUTGGAAUATYGAGGUAGTGUYGGYGGYGGYGAGGGUAUTGAGUUYGGAGYGGGAUAYGUAGUAGATGUUYGYGGGUUTG
GUTGAGTGTGUUAGGYGUTTGTTGATGAGGYGGATTGGGGUAGYGGGAUAUAUTAUYGGGTGGYGGYGGTGGUTUAAYGGUYGGGAGYGGAYGAUUU
YGAUTGGAGGAGGTGUTGGUTUYGYGGUTTGGGGTTYGGGUTYGGUTYGGUTUAGGUAYGUYGGUAGGYGAGUTGGUTUYGUTATTGGGGTGGGYGG
ATTGGYGGYGYGGGYGUUATGGAGAUUUUTGTUAGTUAUYGGGTGGUTUTGUUAATUAAAGUTTGAATUAGUAYGUUAGGAUTATTTUAGUAUUTU
UUYGTGUTYGAUAGYGUUYGUYG

### Example 2. STAMP-EP1: lung cancer cell line model detection-BSP (Bisulfite Sequencing PCR) detection

1. Genomic DNA was extracted from lung cancer cell line A549 and normal lung fibroblast cell line MRC5;
2. The extracted genomic DNA of A549 and MRC5 cell lines were treated with bisulfite and used as templates for subsequent PCR amplification;
3. Primers were designed according to SEQ ID NO: 1. Four pairs of primers were designed for amplification of different sequence regions. The primer sequences and the detected methylation sites are shown in Table 1.

**Table 1**

| Primer Name | 5'-3' Primer Sequence | Detected CpG site No. |
|---|---|---|
| STAMP-EP1-Sanger-1-Primer-F | AGGTTTTGTAAAATATTGAAGAAAAGTTTAGGAG (SEQ ID NO: 5) | CpG sites 001-059 |
| STAMP-EP1-Sanger-1-Primer-R | CAACCTCCTAATAATATACTTCAAACCACAA (SEQ ID NO: 6) | |
| STAMP-EP1-Sanger-2-Primer-F | TATTTTGGAAAATTATAAGTTTATTAAGGAG (SEQ ID NO: 7) | CpG sites 060-100 |
| STAMP-EP1-Sanger-2-Primer-R | CAAAAATTAACTTTAAATCTTAAAATCCCTAAC (SEQ ID NO: 8) | |
| STAMP-EP1-Sanger-3-Primer-F | AATTTTTGTTAGTTTGGGTATAGGTTTTTATTAT (SEQ ID NO: 9) | CpG sites 101-147 |
| STAMP-EP1-Sanger-3-Primer-R | ACCCTATAACAAAACCTACTACTAAAATCTAC (SEQ ID NO: 10) | |
| STAMP-EP1-Sanger-4-Primer-F | GAATTATGGTGGGGTATAATAGTAGGGATT (SEQ ID NO: 11) | CpG sites 148-178 |
| STAMP-EP1-Sanger-4-Primer-R | TCTAAACAAACRCTCAACTTACAATTCRAATTC (SEQ ID NO: 12) | |

4. After PCR amplification, 2% agarose gel electrophoresis was used to detect the specificity of the PCR fragments. The target fragments were recovered by gel cutting and connected to T vector which was transformed into competent *Escherichia coli.* The bacteria were spread on plate, and clones were selected the next day and sequenced. Ten clones were selected for each fragment for Sanger sequencing.
5. The sequencing results are shown in Figure 1. The average methylation value of lung cancer cell line A549 is 70.73%, while that in normal lung fiber cell line MRC5 is 0.45%.
6. The results showed that STAMP-EP1 was an excellent marker for lung cancer.

### Example3.STAMP-EP1:lung cancer - clinical sample assay - pyrosequencing

1. Clinical samples: 20 pairs of paracancerous-lung cancer samples were obtained, with paracancerous samples as control group and lung cancer samples as experimental group;
2. DNA extraction: DNA was extracted from the experimental group and the control group respectively. Phenol-chloroform extraction method was used in this experiment, which is not limited thereto;
3. Bisulfite treatment: the extracted DNA samples were treated with bisulfite and the procedures were strictly followed. EZ DNA Methylation-Gold Kit (ZYMO Research, Cat# D5006) was used in this experiment, which is not limited thereto;
4. PCR amplification: the bisulfite treated samples were used as PCR templates, and PCR primers and pyrosequencing primers were designed based on STAMP-EP1 sequences. Following PCR amplification, the methylation values of STAMP-EP1 were detected by pyrosequencing as the methylation values of sites 064, 065, 066, 067, 068 and 069. The PCR primers sequences, pyrosequencing primers sequences, the detecting sequences of pyrosequencing and the detected sites are shown in Table 2;

**Table 2**

| Primer Name | 5'-3' sequence | Remark |
|---|---|---|
| STAMP-EP1-Pyroseq-Primer-F | GAAGTATATTATTAGGAGGTTGAGAA G (SEQ ID NO: 13) | The sites detected by the primer pair: 064, 065, 066, 067, 068, 069 |
| STAMP-EP1-Pyroseq-Primer-R(5 '-Biotin 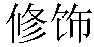 ) | TACRCTCCTTAAAACAATATATCTTC (SEQ ID NO: 14) | |
| STAMP-EP1-Pyroseq-Primer-Seq | CCCTGGGACCTGTGGACAAGT (SEQ ID NO: 15) | |
| Pyrosequencing detecting sequences | ATYGAGTAAGGAAGAAGTTTTYGTTG TYGYGTATTATTTGGGAYGGYG (SEQ ID NO: 16) | |

5. Agarose gel electrophoresis: the specificity of PCR amplification was identified by agarose gel electrophoresis, and the size of the amplified fragment should be 139bp;
6. Pyrosequencing: Pyro Mark Q96 ID pyrosequencing instrument (QIAGEN) was used for sequencing, and the procedures in instructions were strictly followed;
7. Calculation of STAMP-EP1 methylation value: pyrosequencing can detect the methylation values of sites 064, 065, 066, 067, 068, and 069 in the target region, and the average value were calculated as the STAMP-EP1 methylation value in the sample;
8. Results analysis: the methylation value of STAMP-EP1 was compared between the control group and the experimental group, as shown in Figure 2. It showed that among the clinical samples of lung cancer, the STAMP-EP1 methylation value of the lung cancer experimental group was significantly increased, with P < 0.0001, a sensitivity of 100% and a specificity of 100%.

### Example 4. STAMP-EP1: colorectal cancer - clinical sample assay - pyrosequencing

1. Clinical samples: 10 paracancerous clinical samples of colorectal cancer were used as the control group, and 28 colorectal cancer clinical samples were used as the experimental group;
2. Step 2, 3, 4, 5, 6, and 7 are the same as those in Example 3;
3. Results analysis: the methylation value of STAMP-EP1 was compared between the control group and the experimental group, as shown in Figure 3. It showed that among the clinical samples of colorectal cancer, the STAMP-EP1 methylation value of the colorectal cancer experimental group was significantly increased, with P < 0.0001, a sensitivity of 100% and a specificity of 100%.

### Example 5. STAMP-EP1: gastric cancer - clinical sample assay - pyrosequencing

1. Clinical samples: 10 normal gastric (or gastritis) clinical samples were used as the control group, 10 gastric cancer resection edge clinical samples were used as the experimental group 1, and 20 gastric cancer clinical samples were used as experimental group 2;
2. Step 2, 3, 4, 5, 6, and 7 are the same as those in Example 3;
   8. Results analysis: the methylation value of STAMP-EP1 was compared among the normal gastric (or gastritis) control group, the resection edge experimental group 1 and gastric cancer experimental group 2, as shown in Figure 4. The results showed that among the clinical samples of gastric cancer, the STAMP-EP1 methylation value of the gastric cancer experimental group 2 was significantly increased, P < 0.0001. Meanwhile, the methylation of resection edge experimental group 1 was between the normal gastric (or gastritis) control group and the gastric cancer experimental group 2, which indicated that the methylation of STAMP-EP1 began to change at the resection edge. Therefore, as a marker of gastric cancer, STAMP-EP1 can be used to detect gastric cancer, and can also be used to determine the resection edge of gastric cancer.

### Example 6. STAMP-EP1: cervical cancer - clinical sample assay-pyrosequencing

1. Clinical samples: 13 paracancerous clinical samples of cervical cancer were used as the control group, and 26 cervical cancer clinical samples were used as the experimental group;
2. Step 2, 3, 4, 5, 6, and 7 are the same as those in Example 3;
   8. Results analysis: the methylation value of STAMP-EP1 was compared between the control group and the experimental group, as shown in Figure 5. It shows that among the clinical samples of cervical cancer, the STAMP-EP1 methylation value of the cervical cancer experimental group was significantly increased, with P < 0.0001, a sensitivity of 92.3% and a specificity of 100%.

### Example 7. STAMP-EP1: liver cancer - clinical sample assay- pyrosequencing

1. Clinical samples: 21 pairs of paracancerous-liver cancer samples were obtained, with paracancerous samples as liver cancer control group and liver cancer samples as liver cancer experimental group;
2. Step 2, 3, 4, 5, 6, and 7 are the same as those in Example 3;
   8. Results analysis: the methylation value of STAMP-EP1 was compared between the control group and the experimental group, as shown in Figure 6. It shows that among the clinical samples of liver cancer, the STAMP-EP1 methylation value of the liver cancer experimental group was significantly increased, with P < 0.0001, a sensitivity of 100% and a specificity of 100%.

### Example 8. STAMP-EP1: breast cancer - clinical sample assay- pyrosequencing

1. Clinical samples: 22 pairs of paracancerous-breast cancer samples were obtained, with paracancerous samples as control group and breast cancer samples as experimental group;
2. Step 2, 3, 4, 5, 6, and 7 are the same as those in Example 3;
   8. Results analysis: the methylation value of STAMP-EP1 was compared between the control group and the experimental group, as shown in Figure 7. It shows that among the clinical samples of breast cancer, the STAMP-EP1 methylation value of the breast cancer experimental group was significantly increased, with P < 0.0001, a sensitivity of 100% and a specificity of 100%.

### Example 9. STAMP-EP1: pancreatic cancer - clinical sample assay-pyrosequencing

1. Clinical samples: 18 pairs of paracancerous-pancreatic cancer samples were obtained, with paracancerous samples as control group and pancreatic cancer samples as experimental group;
2. Step 2, 3, 4, 5, 6, and 7 are the same as those in Example 3;
   8. Results analysis: the methylation value of STAMP-EP1 was compared between the control group and the experimental group, as shown in Figure 8. It shows that among the clinical samples of pancreatic cancer, the STAMP-EP1 methylation value of the pancreatic cancer experimental group was significantly increased, with P < 0.0001, a sensitivity of 94.44% and a specificity of 100%.

### Example 10. STAMP-EP1: Head and neck cancer - clinical sample assay-pyrosequencing

1. Clinical samples: 11 pairs of paracancerous-head and neck cancer samples were obtained, including 5 cases of laryngeal cancer, 2 cases of tonsil cancer, 2 cases of epiglottis cancer, 1 case of tongue base cancer, and 1 case of hypopharyngeal cancer. Paracancerous samples were used as control group, and cancer samples as experimental group;
2. Step 2, 3, 4, 5, 6, and 7 are the same as those in Example 3;
   8. Results analysis: the methylation value of STAMP-EP1 was compared between the control group and the experimental group, as shown in Figure 9. It shows that among the clinical samples of head and neck cancer, the STAMP-EP1 methylation value of the head and neck cancer experimental group was significantly increased, with P < 0.0001, a sensitivity of 100% and a specificity of 100%.

### Example 11. STAMP-EP1: gallbladder cancer - clinical sample assay-pyrosequencing

1. Clinical samples: bile samples were obtained from 12 non-cancer patients and 10 gallbladder cancer patients, with non-cancer samples as control group and gallbladder cancer samples as experimental group;
2. Step 2, 3, 4, 5, 6, and 7 are the same as those in Example 3;
   8. Results analysis: the methylation value of STAMP-EP1 was compared between the control group and the experimental group, as shown in Figure 10. It shows that among the clinical samples of gallbladder cancer, the STAMP-EP1 methylation value of the gallbladder cancer experimental group was significantly increased, with P < 0.0001, a sensitivity of 90% and a specificity of 100%.

### Example 12. STAMP-EP1: leukemia - clinical sample assay- pyrosequencing

1. Clinical samples: 10 non-leukemia bone marrow smear clinical samples were used as the control group, and 20 leukemia bone marrow smear clinical samples were used as the experimental group;
2. Step 2, 3, 4, 5, 6, and 7 are the same as those in Example 3;
   8. Results analysis: the methylation value of STAMP-EP1 was compared between the control group and the experimental group, as shown in Figure 11. It shows that among the clinical samples of leukemia, the STAMP-EP1 methylation value of the leukemia experimental group was significantly increased, with P < 0.0001, a sensitivity of 100% and a specificity of 100%.

### Example 13. STAMP-EP1: renal cancer - clinical sample assay- pyrosequencing

1. Clinical samples: 8 paracancerous clinical samples of renal cancer were used as the control group, and 16 renal cancer clinical samples were used as the experimental group;
2. Step 2, 3, 4, 5, 6, and 7 are the same as those in Example 3;
   8. Results analysis: the methylation value of STAMP-EP1 was compared between the control group and the experimental group, as shown in Figure 12. It shows that among the clinical samples of renal cancer, the STAMP-EP1 methylation value of the renal cancer experimental group was significantly increased, with P < 0.0001, a sensitivity of 93.75% and a specificity of 100%.

### Example 14. STAMP-EP1: bladder cancer - clinical sample assay-pyrosequencing

1. Clinical samples: 5 cases of bladder cancer paracancerous samples and non-cancer urine samples were used as the control group, and 7 cases of bladder cancer tissue samples and bladder cancer urine samples were used as the experimental group;
2. Step 2, 3, 4, 5, 6, and 7 are the same as those in Example 3;
   8. Results analysis: the methylation value of STAMP-EP1 was compared between the control group and the experimental group, as shown in Figure 13. It shows that among the clinical samples of bladder cancer, the STAMP-EP1 methylation value of the bladder cancer experimental group was significantly increased.

### Example 15. STAMP-EP1: plasma sample - clinical sample assay

1. In order to prove that SATMP-C tumor marker can also be detected using liquid biopsy, 20 normal plasma samples were used as the control group, and plasma samples from patients with different tumor types were obtained, including 10 cases of liver cancer, 10 cases of pancreatic cancer, 10 cases of lung cancer, 10 cases of colorectal cancer, and 10 cases of breast cancer;
2. Step 2, 3, 4, 5, 6, and 7 are the same as those in Example 3;
   8. Results analysis: as shown in Figure 14, compared with the normal plasma control group, the methylation values of liver cancer, pancreatic cancer, lung cancer, colorectal cancer and breast cancer groups were significantly increased.

Each reference provided herein is incorporated by reference to the same extent as if each reference was individually incorporated by reference. In addition, it should be understood that based on the above teaching content of the disclosure, those skilled in the art can practice various changes or modifications to the disclosure, and these equivalent forms also fall within the scope of the appended claims.

## Claims

1. An isolated polynucleotide, wherein the isolated polynucleotide comprises:
(a) the polynucleotide with a nucleotide sequence as shown in SEQ ID NO: 1;
(b) a fragment of the polynucleotide of (a), having at least one CpG site with modification; and/or
(c) a nucleic acid complementary to the polynucleotide or fragment of (a) or (b).

2. The isolated polynucleotide according to claim 1, wherein the modification comprises 5-methylation, 5-hydroxymethylation, 5-aldehyde methylation or 5-carboxymethylation.

3. An isolated polynucleotide, wherein, the polynucleotide is converted from the polynucleotide according to claim 1 or 2, and as compared with the sequence in claim 1, its cytosine C of the CpG site(s) with modification is unchanged, and the unmodified cytosine is converted into T or U.

4. The polynucleotide according to claim 3, wherein the polynucleotide comprises:
(d) the polynucleotide with a nucleotide sequence as shown in SEQ ID NO: 2 or 4;
(e) a fragment of the polynucleotide of (d), having at least one CpG site with modification.

5. Use of the polynucleotide according to any of claims 1-4 in manufacture of a tumor detection agent or kit.

6. The use according to claim 5, wherein the tumors comprise: hematologic cancers such as leukemia, lymphoma, multiple myeloma; digestive system tumors such as esophageal cancer, gastric cancer, colorectal cancer, liver cancer, pancreatic cancer, bile duct and gallbladder cancer; respiratory system tumors such as lung cancer, pleuroma; nervous system tumors such as glioma, neuroblastoma, meningioma; head and neck tumors such as oral cancer, tongue cancer, laryngeal cancer, nasopharyngeal cancer; gynecological and reproductive system tumors such as breast cancer, ovarian cancer, cervical cancer, vulvar cancer, testicular cancer, prostate cancer, penile cancer; urinary system tumors such as kidney cancer, bladder cancer, skin and other systems tumors such as skin cancer, melanoma, osteosarcoma, liposarcoma, thyroid cancer.

7. The use according to claim 5 or 6, wherein samples of the tumor comprise: tissue samples, paraffin embedded samples, blood samples, pleural effusion samples, and alveolar lavage fluid samples, ascites and lavage fluid samples, bile samples, stool samples, urine samples, saliva samples, sputum samples, cerebrospinal fluid samples, cell smear samples, cervical scraping or brushing samples, tissue and cell biopsy samples.

8. A method of preparing a tumor detection agent, comprising: providing the polynucleotide according to any of claims 1-4, designing a detection agent for specifically detecting a target sequence which is the full length or fragment of the polynucleotide; wherein, the target sequence has at least one modified CpG site; preferably, the detection agent comprises: primers or probes.

9. An agent or a combination of agents, wherein the agent or the combination of agents specifically detect the modification on CPG site(s) of a target sequence, which is the full length or fragment of the polynucleotides according to any of claims 1-4 and has at least one modified CpG site.

10. The agent or the combination of agents according to claim 9, wherein the agent or combination of agents is for a gene sequence containing the target sequence, and the gene sequence comprises a gene Panel or a gene group.

11. The agent or the combination of agents according to claim 9, wherein the agent or combination of agents comprise: primers or probes; preferably, the primers are:
the primers shown in SEQ ID NO: 5 and 6;
the primers shown in SEQ ID NO: 7 and 8;
the primers shown in SEQ ID NO: 9 and 10;
the primers shown in SEQ ID NO: 11 and 12; or
the primers shown in SEQ ID NO: 13 and 14.

12. Use of the agent or combination of agents according to any of claims 9-11 in the manufacture of a kit for detecting tumors; preferably, the tumors comprise: digestive system tumors such as esophageal cancer, gastric cancer, colorectal cancer, liver cancer, pancreatic cancer, bile duct and gallbladder cancer; respiratory system tumors such as lung cancer, pleuroma; hematologic cancers such as leukemia, lymphoma, multiple myeloma; gynecological and reproductive system tumors such as breast cancer, ovarian cancer, cervical cancer, vulvar cancer, testicular cancer, prostate cancer, penile cancer; nervous system tumors such as glioma, neuroblastoma, meningioma; head and neck tumors such as oral cancer, tongue cancer, laryngeal cancer, nasopharyngeal cancer; urinary system tumors such as kidney cancer, bladder cancer, skin and other systems tumors such as skin cancer, melanoma, osteosarcoma, liposarcoma, thyroid cancer.

13. A detection kit, comprising:
container(s) and the agent or combination of agents according to any of claims 9-11 in the container(s).

14. A method of detecting the methylation profile of a sample *in vitro,* comprising:
(i) providing the sample and extracting nucleic acid;
(ii) detecting the modification on CPG site(s) of a target sequence in the nucleic acid of (i), wherein the target sequence is the polynucleotide according to claim 1 or 2 or the polynucleotide according to claim 3 or 4 that converted from the polynucleotide according to claim 1 or 2.

15. The method according to claim 14, wherein, in step (3), the analysis methods comprise pyrosequencing, bisulfite conversion sequencing, method using methylation chip, qPCR, digital PCR, second generation sequencing, third generation sequencing, whole genome methylation sequencing, DNA enrichment detection, simplified bisulfite sequencing technology, HPLC, MassArray, methylation specific PCR, or their combination, as well as *in vitro* detection and *in vivo* tracer detection for the combined gene group of partial or all of the methylation sites in the sequence shown in SEQ ID NO: 1.

16. The method according to claim 14, wherein, step (ii) comprises:
(1) treating the product of (i) to convert the unmodified cytosine into uracil;
preferably, the modification includes 5-methylation, 5-hydroxymethylation, 5-aldehyde methylation or 5-carboxymethylation; preferably, treating the nucleic acid of step (i) with bisulfite;
(2) analyzing the modification of the target sequence in the nucleic acid treated by (1).


Sequenceing List

<110>  SHANGHAI EPIPROBE BIOTECHNOLOGY CO.,LTD.

<120>  Methylation Modification-Based Tumor Marker STAMP-EP1

<130>  185625PCWO

<160>  12

<170>  SIPOSequenceListing 1.0

<210>  1
<211>  2448
<212>  DNA
<213>  Homo sapiens

<400>  1
cggcgggcgc tgtcgagcac ggggaggtgc tgaaatagtc ctggcgtgct gattcaagct      60
ttgattggca gagccacccg gtgactgaca gggggtctcc atggcgcccg cgccgccaat     120
ccgcccaccc caatagcgga gccagctcgc ctgccggcgt gcctgagccg agccgagccc     180
gaaccccaag ccgcggagcc agcacctcct ccagtcgggg tcgtccgctc ccggccgttg     240
agccaccgcc gccacccggt agtgtgtccc gctgccccaa tccgcctcat caacaagcgc     300
ctggcacact cagccaggcc cgcgggcatc tgctgcgtgt cccgctccgg gctcagtgcc     360
ctcgccgccg ccggcactgc ctcgatgttc cagctgccca tcttgaattt cagcccccag     420
caagtggccg gggtatgtga gaccctggaa gagagcggcg atgtggagcg cctgggtcgc     480
ttcctctggt cgctgcccgt ggcccctgcg gcctgcgagg ccctcaacaa gaatgagtcg     540
gtgctacgcg cacgagccat cgtggccttt cacggtggca actaccgcga gctctatcat     600
atcctggaaa accacaagtt caccaaggag tcgcacgcca agctgcaggc gctgtggctt     660
gaagcacact accaggaggc tgagaagctg cgtggaagac ccctgggacc tgtggacaag     720
taccgagtaa ggaagaagtt cccgctgccg cgcaccattt gggacggcga acagaagaca     780
cactgcttca aggagcgcac gcggcacctg ctacgcgagt ggtacctgca ggatccatac     840
cctaacccca gcaaaaaacg tgagctcgcc caggcaaccg gactgacccc tacgcaggtg     900
ggcaactggt tcaaaaaccg ccgacaaagg gaccgagcgg ctgcagccaa gaacaggtcg     960
gtacctagag gcctccgcgc tttgagcgca ccggggagga ggcgggtgga ggcacctctg    1020
gcgcccttac ccagtccctg gcgactccaa ttcagcagga gttgggagcg cggtctgtct    1080
tgggttaaga gccctgcgtt ctgggctcct ggccgggagt tcccttgccg gctctgcttc    1140
cccacccgct ggctccccac gcctgcgggc agctgcagca gctggtcccg gtcaccaaac    1200
caaggcttca ctgggacgga gaggggaaga gaaataaaaa attaaaatcc tacaaacagt    1260
tagggacccc aagacccaaa gctaattctt gtcagcctgg gcacaggctc ctactattaa    1320
tcgaagcctg gcttattagc aatgtgtcgg tttcatgtta attatcattt tcaaagccca    1380
ggtatatccc tccctaatgc tttgaaaaca gttttcaatg gacttttgag aaatgggaag    1440
tcgagttttc ctcttcccat gcgctgcctg ccactcttgt ctcaaaacag caaactagtc    1500
cgtgggccga ggcttttcgt ttcccggagt gtggatctcg attagccaaa cattttgcgg     1560
aagagcccgg cctcatcccc caggcccaaa tgctccttac aatccttttt gcctttaggt    1620
cgggccgacc cgatccaacg cgatcgcggg agcacttgct caggcgtaag ccccaggcag    1680
acgcaccgtt agaaatggta tcccatgtcc ctgggaccga tctgtccttg tcacccacac    1740
ttcgtttatt tcctgacagt cctgtaaatc tcccaaaagt gcacaacaaa cagggaggac     1800
actgcaagcc cagtatataa aagacctggg agctgcggcg ctgagaaagg gcgcgaatca    1860
tggtggggca caacagtagg gacccgcgga ggggcggccg cggactcctg cccgacctct    1920
gtcgccttgc cgagtaatcc tcgccttaac tgctggggtc ttcggaagaa cctctagccg    1980
ccgggctgga gggacgcagg aggtggtggg ggcgggcgac gggcggctgt gttacgagct    2040
gtgacccgtg ttccctttct tccccgtaga ctccagcagc aggtcctgtc acagggttcc    2100
gggcgggcac tacgggcgga gggcgacggc acgccagagg tgctgggcgt cgccaccagc    2160
ccggccgcca gtctatccag caaggcggcc acttcagcca tctccatcac gtccagcgac    2220
agcgagtgcg acatctgagt tgcccatcca ggatgctcag aagcagattc cagtgtaaaa    2280
acgagaaaaa caaaatgaaa gaggggaaga agatgagaga cctgcaaatc cagcgccaca    2340
gaagccaggt gaccagggac ccgcgggctc gggttgccgt ttcccgcccc accccgcggc    2400
cggcctggct tcactggcgc cctttggccg cgaccacggg aaccagcg                 2448

<210>  2
<211>  2448
<212>  DNA/RNA

<213>  Artificial Sequence

<220>
<221>  misc_feature
<222>  (1)..(2448)
<223>  bisulfite treated sequence of seq id no:1


<220>
<221>  misc_recomb
<222>  (1)..(2448)
<223>  y is c or u


<400>  2
yggygggygu tgtygaguay ggggaggtgu tgaaatagtu utggygtgut gattuaagut      60
ttgattggua gaguuauuyg gtgautgaua gggggtutuu atggyguuyg yguyguuaat     120
uyguuuauuu uaatagygga guuagutygu utguyggygt guutgaguyg aguygaguuy     180
gaauuuuaag uygyggaguu aguauutuut uuagtygggg tygtuygutu uygguygttg     240
aguuauyguy guuauuyggt agtgtgtuuy gutguuuuaa tuyguutuat uaauaagygu     300
utgguauaut uaguuagguu ygyggguatu tgutgygtgt uuygutuygg gutuagtguu     360
utyguyguyg uygguautgu utygatgttu uagutguuua tuttgaattt uaguuuuuag     420
uaagtgguyg gggtatgtga gauuutggaa gagagyggyg atgtggagyg uutgggtygu     480
ttuututggt ygutguuygt gguuuutgyg guutgygagg uuutuaauaa gaatgagtyg     540
gtgutaygyg uaygaguuat ygtgguuttt uayggtggua autauygyga gututatuat     600
atuutggaaa auuauaagtt uauuaaggag tyguayguua agutguaggy gutgtggutt     660
gaaguauaut auuaggaggu tgagaagutg ygtggaagau uuutgggauu tgtggauaag     720
tauygagtaa ggaagaagtt uuygutguyg yguauuattt gggayggyga auagaagaua     780
uautguttua aggagyguay gygguauutg utaygygagt ggtauutgua ggatuuatau     840
uutaauuuua guaaaaaayg tgagutyguu uagguaauyg gautgauuuu tayguaggtg     900
gguaautggt tuaaaaauyg uygauaaagg gauygagygg utguaguuaa gaauaggtyg     960
gtauutagag guutuygygu tttgagygua uyggggagga ggygggtgga gguauututg    1020
gyguuuttau uuagtuuutg gygautuuaa ttuaguagga gttgggagyg yggtutgtut    1080
tgggttaaga guuutgygtt utgggutuut gguygggagt tuuuttguyg gututguttu    1140
uuuauuygut ggutuuuuay guutgygggu agutguagua gutggtuuyg gtuauuaaau    1200
uaagguttua utgggaygga gaggggaaga gaaataaaaa attaaaatuu tauaaauagt    1260
tagggauuuu aagauuuaaa gutaattutt gtuaguutgg guauaggutu utautattaa    1320
tygaaguutg guttattagu aatgtgtygg tttuatgtta attatuattt tuaaaguuua    1380
ggtatatuuu tuuutaatgu tttgaaaaua gttttuaatg gauttttgag aaatgggaag    1440
tygagttttu ututtuuuat gygutguutg uuaututtgt utuaaaauag uaaautagtu    1500
ygtggguyga gguttttygt ttuuyggagt gtggatutyg attaguuaaa uattttgygg    1560
aagaguuygg uutuatuuuu uagguuuaaa tgutuuttau aatuuttttt guutttaggt    1620
yggguygauu ygatuuaayg ygatygyggg aguauttgut uaggygtaag uuuuagguag    1680
ayguauygtt agaaatggta tuuuatgtuu utgggauyga tutgtuuttg tuauuuauau    1740
ttygtttatt tuutgauagt uutgtaaatu tuuuaaaagt guauaauaaa uagggaggau    1800
autguaaguu uagtatataa aagauutggg agutgyggyg utgagaaagg gygygaatua    1860
tggtggggua uaauagtagg gauuygygga ggggygguyg yggautuutg uuygauutut    1920
gtyguuttgu ygagtaatuu tyguuttaau tgutggggtu ttyggaagaa uututaguyg    1980
uygggutgga gggayguagg aggtggtggg ggygggygay gggyggutgt gttaygagut    2040
gtgauuygtg ttuuutttut tuuuygtaga utuuaguagu aggtuutgtu auagggttuy    2100
gggyggguau taygggygga gggygayggu ayguuagagg tgutgggygt yguuauuagu    2160
uygguyguua gtutatuuag uaaggygguu auttuaguua tutuuatuay gtuuagygau    2220
agygagtgyg auatutgagt tguuuatuua ggatgutuag aaguagattu uagtgtaaaa    2280
aygagaaaaa uaaaatgaaa gaggggaaga agatgagaga uutguaaatu uagyguuaua    2340
gaaguuaggt gauuagggau uygyggguty gggttguygt ttuuyguuuu auuuygyggu    2400
ygguutggut tuautggygu uutttgguyg ygauuayggg aauuagyg                 2448

<210>  3
<211>  2448
<212>  DNA
<213>  Homo sapiens

<400>  3

cgctggttcc cgtggtcgcg gccaaagggc gccagtgaag ccaggccggc cgcggggtgg 60
ggcgggaaac ggcaacccga gcccgcgggt ccctggtcac ctggcttctg tggcgctgga 120
tttgcaggtc tctcatcttc ttcccctctt tcattttgtt tttctcgttt ttacactgga 180
atctgcttct gagcatcctg gatgggcaac tcagatgtcg cactcgctgt cgctggacgt 240
gatggagatg gctgaagtgg ccgccttgct ggatagactg gcggccgggc tggtggcgac 300
gcccagcacc tctggcgtgc cgtcgccctc cgcccgtagt gcccgcccgg aaccctgtga 360
caggacctgc tgctggagtc tacggggaag aaagggaaca cggtcacagc ctcgtaacac 420
agccgcccgt cgcccgcccc caccacctcc tgcgtccctc cagcccggcg gctagaggtt 480
cttccgaaga ccccagcagt taaggcgagg attactcggc aaggcgacag aggtcgggca 540
ggagtccgcg gccgcccctc cgcgggtccc tactgttgtg ccccaccatg attcgcgccc 600
tttctcagcg ccgcagctcc caggtctttt atatactggg cttgcagtgt cctccctgtt 660
tgttgtgcac ttttgggaga tttacaggac tgtcaggaaa taaacgaagt gtgggtgaca 720
aggacagatc ggtcccaggg acatgggata ccatttctaa cggtgcgtct gcctggggct 780
tacgcctgag caagtgctcc cgcgatcgcg ttggatcggg tcggcccgac ctaaaggcaa 840
aaaggattgt aaggagcatt tgggcctggg ggatgaggcc gggctcttcc gcaaaatgtt 900
tggctaatcg agatccacac tccgggaaac gaaaagcctc ggcccacgga ctagtttgct 960
gttttgagac aagagtggca ggcagcgcat gggaagagga aaactcgact tcccatttct 1020
caaaagtcca ttgaaaactg ttttcaaagc attagggagg gatatacctg ggctttgaaa 1080
atgataatta acatgaaacc gacacattgc taataagcca ggcttcgatt aatagtagga 1140
gcctgtgccc aggctgacaa gaattagctt tgggtcttgg ggtccctaac tgtttgtagg 1200
attttaattt tttatttctc ttcccctctc cgtcccagtg aagccttggt ttggtgaccg 1260
ggaccagctg ctgcagctgc ccgcaggcgt ggggagccag cgggtgggga agcagagccg 1320
gcaagggaac tcccggccag gagcccagaa cgcagggctc ttaacccaag acagaccgcg 1380
ctcccaactc ctgctgaatt ggagtcgcca gggactgggt aagggcgcca gaggtgcctc 1440
cacccgcctc ctccccggtg cgctcaaagc gcggaggcct ctaggtaccg acctgttctt 1500
ggctgcagcc gctcggtccc tttgtcggcg gtttttgaac cagttgccca cctgcgtagg 1560
ggtcagtccg gttgcctggg cgagctcacg ttttttgctg gggttagggt atggatcctg 1620
caggtaccac tcgcgtagca ggtgccgcgt gcgctccttg aagcagtgtg tcttctgttc 1680
gccgtcccaa atggtgcgcg gcagcgggaa cttcttcctt actcggtact tgtccacagg 1740
tcccaggggt cttccacgca gcttctcagc ctcctggtag tgtgcttcaa gccacagcgc 1800
ctgcagcttg gcgtgcgact ccttggtgaa cttgtggttt tccaggatat gatagagctc 1860
gcggtagttg ccaccgtgaa aggccacgat ggctcgtgcg cgtagcaccg actcattctt 1920
gttgagggcc tcgcaggccg caggggccac gggcagcgac cagaggaagc gacccaggcg 1980
ctccacatcg ccgctctctt ccagggtctc acataccccg gccacttgct gggggctgaa 2040
attcaagatg ggcagctgga acatcgaggc agtgccggcg gcggcgaggg cactgagccc 2100
ggagcgggac acgcagcaga tgcccgcggg cctggctgag tgtgccaggc gcttgttgat 2160
gaggcggatt ggggcagcgg gacacactac cgggtggcgg cggtggctca acggccggga 2220
gcggacgacc ccgactggag gaggtgctgg ctccgcggct tggggttcgg gctcggctcg 2280
gctcaggcac gccggcaggc gagctggctc cgctattggg gtgggcggat tggcggcgcg 2340
ggcgccatgg agaccccctg tcagtcaccg ggtggctctg ccaatcaaag cttgaatcag 2400
cacgccagga ctatttcagc acctccccgt gctcgacagc gcccgccg 2448

<210> 4
<211> 2448
<212> DNA/RNA
<213> Artificial Sequence

<220>
<221> misc_feature
<222> (1)..(2448)
<223> bisulfite treated sequence of seq id no:3


<220>
<221> misc_feature
<222> (1)..(2448)
<223> y is c or u


<400> 4
ygutggttuu ygtggtygyg guuaaagggy guuagtgaag uuagguyggu ygyggggtgg 60
ggygggaaay gguaauuyga guuygygggt uuutggtuau utgguttutg tggygutgga 120
tttguaggtu tutuatuttu ttuuuututt tuattttgtt tttutygttt ttauautgga 180

atutguttut gaguatuutg gatggguaau tuagatgtyg uautygutgt ygutggaygt 240
gatggagatg gutgaagtgg uyguuttgut ggatagautg gygguygggu tggtggygay 300
guuuaguauu tutggygtgu ygtyguuutu yguuygtagt guuyguuygg aauuutgtga 360
uaggauutgu tgutggagtu tayggggaag aaagggaaua ygggtuauag utygtaauau 420
aguyguuygt yguuyguuuu uauuauutuu tgygtuuutu uaguuyggyg gutagaggtt 480
uttuygaaga uuuuaguagt taaggygagg attautyggu aaggygauag aggtygggua 540
ggagtuygyg guyguuuutu ygygggtuuu tautgttgtg uuuuauuatg attygyguuu 600
tttutuagyg uyguagutuu uaggtutttt atatautggg uttguagtgt uutuuutgtt 660
tgttgtguau ttttgggaga tttauaggau tgtuaggaaa taaaygaagt gtgggtgaua 720
aggauagaty ggtuuuaggg auatgggata uuatttutaa yggtgygtut guutggggut 780
tayguutgag uaagtgutuu ygygatygyg ttggatyggg tygguuygau utaaagguaa 840
aaaggattgt aaggaguatt tggguutggg ggatgagguy gggututtuy guaaaatgtt 900
tggutaatyg agatuuauau tuygggaaay gaaaaguuty gguuuaygga utagtttgut 960
gttttgagau aagagtggua gguagyguat gggaagagga aaautygaut tuuuatttut 1020
uaaaagtuua ttgaaaautg ttttuaaagu attagggagg gatatauutg ggutttgaaa 1080
atgataatta auatgaaauy gauauattgu taataaguua gguttygatt aatagtagga 1140
guutgtguuu aggutgauaa gaattagutt tgggtuttgg ggtuuutaau tgtttgtagg 1200
attttaattt tttatttutu ttuuuututu ygtuuuagtg aaguuttggt ttggtgauyg 1260
ggauuagutg utguagutgu uyguaggygt ggggaguuag ygggtgggga aguagaguyg 1320
guaagggaau tuuygguuag gaguuuagaa yguagggutu ttaauuuaag auagauygyg 1380
utuuuaautu utgutgaatt ggagtyguua gggautgggt aagggyguua gaggtguutu 1440
uauuyguutu utuuuyggtg ygutuaaagy gyggagguut utaggtauyg auutgttutt 1500
ggutguaguy gutyggtuuu tttgtyggyg gtttttgaau uagttguuua uutgygtagg 1560
ggtuagtuyg gttguutggg ygagutuayg ttttttgutg gggttagggt atggatuutg 1620
uaggtauuau tygygtagua ggtguygygt gygutuuttg aaguagtgtg tuttutgtty 1680
guygtuuuaa atggtgygyg guagygggaa uttuttuutt autyggtaut tgtuuauagg 1740
tuuuaggggt uttuuaygua guttutuagu utuutggtag tgtguttuaa guuauagygu 1800
utguaguttg gygtgygaut uuttggtgaa uttgtggttt tuuaggatat gatagaguty 1860
gyggtagttg uuauygtgaa agguuaygat ggutygtgyg ygtaguauyg autuattutt 1920
gttgaggguu tyguagguyg uagggguuay ggguagygau uagaggaagy gauuuaggyg 1980
utuuauatyg uygutututt uuagggtutu auatauuuyg guuauttgut gggggutgaa 2040
attuaagatg gguagutgga auatygaggu agtguyggyg gyggygaggg uautgaguuy 2100
ggagygggau ayguaguaga tguuygyggg uutggutgag tgtguuaggy guttgttgat 2160
gaggyggatt gggguagygg gauauautau ygggtggygg yggtggutua aygguyggga 2220
gyggaygauu uygautggag gaggtgutgg utuygyggut tggggttygg gutyggutyg 2280
gutuagguay guygguaggy gagutggutu ygutattggg gtgggyggat tggyggygyg 2340
ggyguuatgg agauuuuutg tuagtuauyg ggtggututg uuaatuaaag uttgaatuag 2400
uayguuagga utatttuagu auutuuuygt gutygauagy guuyguyg 2448

<210> 5
<211> 34
<212> DNA
<213> Primer

<400> 5
aggttttgta aaatattgaa gaaaagttta ggag 34

<210> 6
<211> 31
<212> DNA
<213> Primer

<400> 6
caacctccta ataatatact tcaaaccaca a 31

<210> 7
<211> 31
<212> DNA
<213> Primer

<400> 7
tattttggaa aattataagt ttattaagga g 31

<210>  8
<211>  33
<212>  DNA
<213>  Primer

<400>  8
caaaaattaa ctttaaatct taaaatccct aac                                    33

<210>  9
<211>  34
<212>  DNA
<213>  Primer

<400>  9
aatttttgtt agtttgggta taggttttta ttat                                   34

<210>  10
<211>  32
<212>  DNA
<213>  Primer

<400>  10
accctataac aaaacctact actaaaatct ac                                     32

<210>  11
<211>  30
<212>  DNA
<213>  Primer

<400>  11
gaattatggt ggggtataat agtagggatt                                        30

<210>  12
<211>  33
<212>  DNA
<213>  Primer

<400>  12
tctaaacaaa crctcaactt acaattcraa ttc                                    33